Europäisches Patentamt

European Patent Office

Office européen des brevets

(11) Publication number : **0 419 251 B1**

(12) **EUROPEAN PATENT SPECIFICATION**

(45) Date of publication of patent specification :
01.09.93 Bulletin 93/35

(51) Int. Cl.⁵ : **A61K 47/26, A61K 37/54**

(21) Application number : **90310287.9**

(22) Date of filing : **20.09.90**

(54) **Protein-containing aqueous solutions.**

(30) Priority : **21.09.89 JP 243311/89**

(43) Date of publication of application :
**27.03.91 Bulletin 91/13**

(45) Publication of the grant of the patent :
**01.09.93 Bulletin 93/35**

(84) Designated Contracting States :
**AT BE CH DE DK ES FR GB GR IT LI LU NL SE**

(56) References cited :
**EP-A- 0 156 169**
**EP-A- 0 198 321**
**EP-A- 0 268 110**
**GB-A- 2 107 185**
**US-A- 4 857 320**

(73) Proprietor : **MITSUI TOATSU CHEMICALS, Inc.**
**2-5 Kasumigaseki 3-chome**
**Chiyoda-Ku Tokyo 100 (JP)**
Proprietor : **MOCHIDA PHARMACEUTICAL CO., LTD.**
**7, Yotsuya 1-chome**
**Shinjuku-ku Tokyo 160 (JP)**

(72) Inventor : **Shimazaki, Yukio**
**45, Fujimi Apaato, 2225-1, Tougou**
**Mobara-shi, Chiba-ken (JP)**
Inventor : **Kawashima, Nobuhiro**
**355., Tounodai Shataku, 90-1, Machibo**
**Mobara-shi, Chiba-ken (JP)**
Inventor : **Yoshioka, Miki**
**141, Mutsunoryo, 2785-1, Mutsuno**
**Mobara-shi, Chiba-ken (JP)**
Inventor : **Tanaka, Yasuhito**
**640-36, Miyanoda Apaato, 2141, Tougou**
**Mobara-shi, Chiba-ken (JP)**
Inventor : **Tanaka, Ryo,**
**3-7-2, Seko,**
**Fujieda-shi, Shizuoka-ken, (JP)**
Inventor : **Sakai, Kiyoshi,**
**1-10-15, Takasu,**
**Fujieda-shi, Shizuoka-ken, (JP)**
Inventor : **Ishiwari, Hisahiro,**
**117-22, Kamiyabuta,**
**Fujieda-shi, Shizuoka-ken, (JP)**

(74) Representative : **Hutchins, Michael Richard et al**
**Graham Watt & Co. London Road Riverhead**
**Sevenoaks, Kent TN13 2BN (GB)**

EP 0 419 251 B1

## Description

Background of the Invention

1. Field of the Invention

The present invention relates to protein-containing aqueous solutions, methods for increasing the protein concentration and a protein preparation and to techniques applicable in the preparation of pharmaceuticals for clinical use using physiologically active proteins.

2. Description of the Prior Art

In using a protein as a homogeneous component, it is extremely important to dissolve the protein in a solvent. For example, where a certain amount of a protein is fractionated from a composition which contains that protein or where analysis on the protein is made, it has to be guaranteed that the composition containing the protein is homogeneous. Furthermore, when a protein is dissolved in water for administration as a pharmaceutical such as an injectable preparation, the protein has to be completely dissolved.

In general, the solubility of proteins in an aqueous solvent is strongly affected by hydrophilic or hydrophobic residues present on the surface of the protein and by charges on the protein. When the protein is only slightly dissolved because of the presence of hydrophobic residues on the surface of the protein, it is possible to increase the solubility by adding a surfactant.

On the other hand, when the pH of an aqueous solvent is near the isoelectric point of the protein to be dissolved, which readily causes the isoelectric precipitation, solubility of the protein can be increased by increasing the salt concentration and the ionic strength of the aqueous solvent. In this case, a surfactant does not contribute to the increase of the protein solubility. Furthermore, when the pH of an aqueous solvent is near the isoelectric point of a protein and the salt concentration is low, the protein is soluble only at a relatively low concentration. Therefore, in order to dissolve the protein in a relatively high concentration, either a method in which a pH separate from the isoelectric point is used or a method in which the salt concentration is increased is generally used.

However, in some cases, it is necessary to dissolve a protein at a sufficiently high concentration without increasing the salt concentration at the pH near the isoelectric point. An example is when a physiologically active protein having the isoelectric point near neutral pH is administered, in a form of a solution at a pH near neutral, to a patient who should maintain his or her salt intake as as low as possible. In this case, the only possible techniques hitherto been either to use the protein in a lower concentration or to administer salt. Thus there were serious practical problems to be solved in the formulation of protein-containing active pharmaceuticals.

Summary of the Invention

An object of the present invention is to provide a highly concentrated aqueous protein solution. Another object of the present invention is to provide a method in which a protein can be dissolved at a high concentration in an aqueous solution. Another object of the present invention is to provide a protein preparation having excellent solubility.

In preparing concentrated protein-containing solutions, the present inventors first found that when a protein is bound to an ion exchanger at an appropriate pH, the binding of the protein to the ion exchanger is apt to occur at a low salt concentration, and that it is thus possible to dissolve a protein by substituting hydrophobic residues of a surfactant used to dissolve a hydrophobic protein with anionic residues, based on the same mechanism as when a surfactant is used.

Based on this finding, the present inventors additionally determined in the course of further investigations that anionic polymers or salts of anionic polymers exert the favourable effect mentioned above, and thus completed the present invention.

The present invention provides a protein-containing aqueous solution in which an anionic polymer or a salt thereof coexists, a method for increasing the protein concentration of an aqueous solution containing a protein in which an anionic polymer or salt thereof coexists, and a preparation containing an anionic polymer or a salt thereof and a protein.

A pharmaceutical preparation containing a saccharide, a type of anionic polymer, such as polysulfate or a sulfonated sugar and t-PA (tissue-type plasminogen activator) has been disclosed in Japanese Patent Laid-open No. 236730/1986. However, the disclosed technique is to improve t-PA activity and is fundamentally different, in terms of technological idea, from that of the present invention which more broadly is to improve the solubility of proteins in general.

The present invention provides a protein-containing aqueous solution at high protein concentrations that conventional techniques have never been able to achieve, especially protein-containing aqueous solutions having a low salt concentration at a pH near the isoelectric point.

Conventionally, in order to dissolve a protein at a pH near the protein's isoelectric point, it is essential to significantly (1) decrease the protein concentration or (2) increase the salt concentration. The resulting protein solution is extremely inconvenient when a physiologically active protein has to be administered in a form of a solution at a pH near the isoelectric point of the protein to a patient whose salt intake is restricted. In order to dissolve the protein without increasing the salt concentration, it is essential to select a pH separate from the pH near the isoelectric point of the protein even if the selected pH is undesirable for the use in pharmaceuticals such as injections or other parenteral dosage forms.

It is known in the art, as reported in US-A-4,857,320, that the protein t-Pa is not readily soluble in aqueous media. Various attempts had hitherto been made to improve its solubility, amongst which are mentioned the use of TWEEN[R]80 plus the expedient of raising the ionic concentration of NaCl saline. Further to enhance the solubility of t-Pa, US-A-4,857,320 teaches that arginine or a non-toxic salt of arginine should be added to the TWEEN[R]80 - containing saline.

By contrast, according to the present invention, proteins can be dissolved without increasing the salt concentration at a pH near the isoelectric point of the protein so that a protein-containing aqueous solution with a low salt concentration at a pH near the isoelectric point of the protein can be provided. The present invention provides an advantageous technique as compared to conventional ones.

The present invention is particularly suitable for preparing low salt pharmaceutical preparations for injection of a physiologically active protein having an isoelectric point near neutral.

According to the present invention, there is provided an aqueous solution of an increased protein concentration comprising said protein and an anionic polymer or a salt thereof, the concentration of said protein in said solution being greater than the maximum concentration in the absence of said anionic polymer, and said solution having a pH in the range within ±2 pH units of the isoelectric point of said protein and an ionic strength of 0.05 mole/l or less.

The invention also provides a method for increasing the concentration of protein in an aqueous solution comprising adding an anionic polymer or salt thereof to said solution, wherein the amount of said anionic polymer is sufficient to dissolve the protein to a concentration greater than the maximum concentration attainable in the absence of anionic polymer, and said solution has a pH within the range of ±2 pH units of the isoelectric point of the protein and an ionic strength of 0.05 mole/l or less.

Still further, the invention provides a pharmaceutical composition comprising an anionic polymer or salt thereof and a protein having poor solubility at a pH range around its isoelectric point, wherein the amount of said anionic polymer is sufficient to obtain an aqueous solution containing a greater amount of protein than the maximum amount of protein attainable in the absence of anionic polymer, the solution having a pH within the range of ±2 pH units of the isoelectric point of the protein and an ionic strength of 0.05 mole/l or less.

Detailed Description and the Preferred Embodiments

Examples of proteins to be used in the present invention include physicochemically simple proteins, conjugated proteins and induced proteins as well as proteins having relatively large amounts of hydrophobic groups. In particular, this invention is suitably applied to proteins such as globulin or t-PA which tend to drastically decrease their solubility at a pH ranging around the isoelectric points. Examples include proteins having an isoelectric point apart from the extremely acid region, preferably pH 4 or higher, and desirably a pH 5 or higher. These proteins may be obtained by extraction and purification from naturally occurring biological bodies or parts thereof, by chemical synthesis or by using genetic recombinant DNA techniques from cell culture. The protein obtained as mentioned above can be used after modification. Examples of these proteins include gamma-globulins such as immunoglobulins A, G and E, lactoglobulin, urokinase, pro-urokinase and tissue-type plasminogen activator (t-PA). In particular, the present invention is specifically adapted for proteins having physiological activities.

The proteins can be used alone or as a mixture of two or more proteins or different types of proteins.

Examples of anion residues of anionic polymers used in the present invention include carboxyl, carboxymethyl, sulfuric and phosphoric groups. Examples of polymer backbones of the anionic polymers include sugars such as sugar alcohol, cellulose, amylose, amino acids and nucleic acid bases, preferably those having a molecular weight of 1,000 - 1,000,000 as the anionic polymer. Examples of the anionic polymers having both anion residues and polymer backbones are the carboxymethyl-ion-exchangers such as carboxymethylamy-

EP 0 419 251 B1

lose and carboxymethylcellulose, acidic polysaccharides such as arginic acid, mucosaccharide sulfates such as dextran sulfate, chondroitin sulfate, chondroitin sulfate A, chondroitin sulfate B, chondroitin sulfate C, chondroitin sulfate D, chondroitin sulfate E, heparin, kerato sulfate, keratane sulfate and heparitine sulfate, acidic poly-amino-acids such as poly-L-glutamic acid and nucleic acids. Illustrative salts of these anionic polymers include sodium, potassium and calcium.

These anionic polymers can be used alone or in combination using two or more types.

An amount of anionic polymer or salt thereof is preferably 1/40 or more (w/w), and desirably 1/10 or more and 100 or less, of that of a protein to be dissolved. If the amount of anionic polymer is not sufficient, the desired amount of protein dissolution may not be achieved; if the amount is excessive, the relative amount of protein to be dissolved is decreased, which prevents significant use of the protein-containing aqueous solution. Since anionic polymers and salts thereof are different from one another in the type of anionic residues, capability in exchanging cations, molecular weights and the like, the amounts of the anionic polymers and the salts thereof may be determined according to the physical properties of the protein to be formulated.

The concentration of the protein in a protein-containing aqueous solution of the present invention is determined according to the solubility of the protein to be dissolved and is unique to each protein and thus cannot be generalized. However, it is generally possible to obtain an aqueous solution having a protein concentration of 0.1 mg/ml or more since the solubility, particularly in the range of pHs near the isoelectric point, is highly improved as compared to that conventionally attained in corresponding protein-containing aqueous solutions. According to the invention, it is normally possible to prepare aqueous protein solution having protein concentration of approximately 0.01 to 10 mg/ml.

In the present invention, the pH range of the aqueous solution is not particularly restricted. However, the present invention is characterized in that the solubility of the protein can be increased at a pH near the isoelectric point of the protein without increasing a salt concentration. Considering the fact that the isoelectric points of proteins are mostly in the range between weakly acidic and alkaline pHs, the invention is particularly significant when the pH of the aqueous solution is in the range of weakly acidic, neutral, weakly alkaline or alkaline pHs. Specifically, the pH of the aqueous solution is within the range of -2 to +2, preferably -1 to +1, pH units away from the isoelectric point of the protein.

In the present invention, the isoelectric point of the protein to be formulated is that determined by electrophoresis. Further, in the case of protein, the isoelectric point is occasionally not converged into a point but demonstrated as a band of a certain range of pH when determined by electrophoresis; in such a case, the pH range denotes the isoelectric point. Further, in a mixture of more than two proteins each having different isoelectric points, a pH range which covers isoelectric points of all of the proteins denotes the isoelectric point of the protein mixture.

The ionic strength of an aqueous solution containing a protein and an anionic polymer or a salt thereof according to the present invention is 0.05 mol/l or less, more preferably 0.02 mol/l or less, particularly preferably 0.01 mol/l or less. If the ionic strength exceeds 0.05 mol/l - the effect of the invention is readily lessened. This is probably because an interaction between the anionic residues of the anionic polymer and the protein is counteracted in the solution with high ionic strength.

Furthermore, the content of salts other than the anionic polymer or a salt thereof is preferably less than 0.1 mol per 1 mg protein. For example, in a solution with a high sodium chloride content, the effect due to the anionic polymer may be blocked.

In order to carry out the dissolving procedure of the present invention, the following method may be applied. First, a protein is dissolved at a pH apart from the isoelectric point and then to the resultant protein solution is added a solution containing an anionic polymer or its salt and the pH is adjusted to around the isoelectric point so as to prepare a solution in which the protein and the anionic polymer or the salt thereof coexists. Furthermore, another applicable method is one in which an aqueous solution containing a protein and an anionic polymer or a salt thereof is prepared in advance at a pH apart from the isoelectric point of the protein and then the pH of the solution is readjusted near the isoelectric point of the protein.

In yet another method, a preparation containing a protein and an anionic polymer is prepared and then the preparation is dissolved. In order to produce such a preparation, any conventional method may be used. For example, a protein is dissolved in a diluted solution at a pH apart from the isoelectric point and then an anionic polymer aqueous solution is added to the solution. After pH adjustment, an excipient and the like are added to the solution and the resultant solution is filtered and dispensed into vials to be lyophilized to prepare a pharmaceutical preparation for injection. Alternatively, to a protein aqueous solution is added an anionic polymer so as to form a composite of the protein and the anionic polymer. The resulting composite is precipitated from the solution by adjusting the pH to the isoelectric point of the composite, dried and then dispensed into vials after adding formulation additives so as to give a preparation. Protein contents of the preparations are normally 0.01 to 50 wt%.

4

If necessary, in order to prevent polymerization of the proteins and their adhesion to the containers, a surfactant such as Tween 80, a chelating agent such as EDTA (to eliminate the effect of metal ions on the protein), an agent to stabilize physiologically activity proteins, and furthermore an excipient such as mannitol and lactose (effective when used in a lyophilized preparation) may be added, besides an anionic polymer or a salt thereof, to a protein solution or a lyophilized product.

According to the present invention, a protein can be effectively dissolved without increasing the salt concentration or ionic strength at a pH near the isoelectric point. Conventional techniques have never been able to attain this. It is beyond the common knowledge of conventional dissolution techniques and is considered to be a significant advance in the art. The mechanism of the dissolution effect is not entirely evident; however, it is assumed that when an anionic polymer or a salt thereof is added to a solution at a pH near the isoelectric point of a protein, in which the solubility of the protein is extremely low by itself, the protein and the anionic polymer interact, particularly at low ionic strength, which results in an extreme increase in the solubility.

EXAMPLES

The present invention will be described more specifically in the following Examples.

Example 1

The protein used was human-derived tissue-type plasminogen activator (t-PA), which was obtained by expressing the structural gene of t-PA in cell culture using gene recombinant techniques then purifying and concentrating it from the culture fluid. The t-PA had been dissolved in a 60 mM sodium phosphate solution which was considered to be a sodium dihydrogen phosphate from its pH (4.2). 1 mg of t-PA and 0 - 1 mg of the calcium salt of heparin were placed in a dialysis tube made of cellulose and then the total volume was made up to 1.0 ml with distilled water. Dialysis was carried out against 1,000 ml of a 1mM citrate buffer solution for 3 hours. The fluid in the dialysis tube was transferred to a small polypropylene test tube, centrifuged at 15,000 rpm for 10 minutes and then the solubility of t-PA was determined by measuring the absorption at 280 nm of the supernatent. The isoelectric point of t-PA is about 6.5 - 7.5. The results are shown in Table 1 from which it is evident that the solubility of t-PA was improved by adding calcium heparin, particularly at pHs near the isoelectric point.

## Table 1

| pH | Heparin(calcium salt)added [a] (mg) | | |
|---|---|---|---|
| | 0 | 0.04 | 1.0 |
| 4.0 | 0.95 | – | 0.10 |
| 4.8 | 0.33 | – | 0.43 |
| 5.5 | 0.03 | – | 0.56 |
| 6.0 | 0.02 | – | 0.99 |
| 7.0[b] | 0.04 | – | 0.99 |
| 7.5[b] | 0.01 | 0.89 | – |
| 8.0 | 0.03 | 0.86 | – |

Entries are solubility of t-PA (mg/ml)

a) Amount added per 1 mg of t-PA

b) pH corresponding to the isoelectric point of t-PA

Example 2

The solubility of t-PA was examined in the same manner as described in Example 1, except that various anionic polymers and cationic polymers shown in Table 2 were used in place of heparin in amounts of 0.1 - 0.2 mg. The pH of the solution was adjusted to about 7. The results are given in Table 2.

The solubility of t-PA was drastically increased in all cases with anionic polymers; however, no increase in solubility was observed with any of the cationic polymers. Furthermore, sufficient solubility was not attained even with the addition of sodium chloride at high concentrations.

EP 0 419 251 B1

Table 2

| Additives | | Solubility (mg/ml) |
|---|---|---|
| Name | Amount (mg/mg t-PA) | |
| None | 0 | 0 |
| Anionic polymer | | |
| Poly-L-glutamic acid (sodium salt) | 0.1 | 1.02 |
| DNA (bacterial origin) | 0.18 | 0.99 |
| Dextran sulfate (calcium salt) | 0.1 | 1.03 |
| Heparin (calcium salt) | 0.1 | 1.04 |
| Chondroitin sulfate A (sodium salt) | 0.1 | 1.00 |
| Chondroitin sulfate B (sodium salt) | 0.1 | 1.01 |
| Chondroitin sulfate C (sodium salt) | 0.1 | 1.04 |
| Sodium arginic acid | 0.1 | 1.03 |
| Cationic polymer | | |
| Protamine sulfate | 0.1 | 0.00 |
| Poly-L-lysine | 0.1 | 0.01 |
| Salt | | |
| Sodium chloride | (200 mM) | 0.31 |

Example 3

The solubility of t-PA was examined in the same manner as described in Example 1, except that chondroitin sulfate A and heparan sulfate in amounts shown in Table 3 were used at pH about 7 in place of heparin. The results are shown in Table 3.

The study showed that t-PA solubility was improved particularly when the amount of chondroitin sulfate A and heparan sulfate were 1/40 or more of the t-PA by weight.

7

Table 3

| Additive | Amount added (Ratio to t-PA by weight) | Solubility (mg/ml) |
|---|---|---|
| Chondroitin sulfate A (sodium salt) | 0 | 0.00 |
| | 1/40 | 0.51 |
| | 1/20 | 1.02 |
| | 3/40 | 1.03 |
| | 1/10 | 1.00 |
| Heparan sulfate (sodium salt) | 0 | 0.00 |
| | 1/40 | 0.20 |
| | 1/20 | 1.06 |
| | 3/40 | 1.05 |
| | 1/10 | 1.05 |

Example 4

The pHs of aqueous solutions were adjusted to the isoelectric point of the individual proteins (t-PA and betalactoglobulin) and the solubilities of the proteins were examined with or without the addition of sodium chondroitin sulfate, the amount of which was one tenth of each protein. The results are shown in Table 4.

Table 4

| Protein | t-PA | Beta-lactoglobulin |
|---|---|---|
| Isoelectric points | 6.5 - 7.5 | 5.1 |
| pH of solution | 7 | 5 |
| Solubility (mg/ml) | | |
| Chondroitin sulfate - | 0.02 | <1.06 |
| Chondroitin sulfate + | 0.8 | 5.8 |

Example 5

t-PA                          100 mg
Sodium chondroitin sulfate    10 mg

Lactose                                  500 mg

The above ingredients were dissolved in 25 ml of a 5 mM phosphate buffer solution (pH 7.0). After sterilization by filtration, the resultant solution was dispensed into vials, 2.5 ml each, and then lyophilized to prepare a t-PA preparation. This t-PA preparation could be re-dissolved in a 5% glucose infusion or distilled water for injection.

Example 6

t-PA                                     100 mg
Heparin(sodium salt)                     10 mg
Lactose                                  500 mg

The above ingredients were dissolved in 25 ml of a 5 mM phosphate buffer solution (pH 7.0) . After sterilization by filtration, the resultant solution was dispensed into vials, 2.5 ml each, and then lyophilized to prepare a t-PA preparation. This t-PA preparation could be re-dissolved in a 5% glucose infusion or distilled water for injection.

Example 7

t-PA                                     100 mg
Dextrin sulfate (sodium salt)            10 mg
Lactose                                  500 mg

The above ingredients were dissolved in 25 ml of a 5 mM phosphate buffer solution (pH 7.0). After sterilization by filtration, the resultant solution was dispensed into vials, 2.5 ml each, and then lyophilized to prepare a t-PA preparation. This t-PA preparation could be re-dissolved in a 5% glucose infusion or distilled water for injection.

## Claims

**Claims for the following Contracting States : AT, BE, CH, DE, DK, FR, GB, IT, LI, LU, NL, SE**

1.   An aqueous solution of an increased protein concentration comprising said protein and an anionic polymer or a salt thereof, the concentration of said protein in said solution being greater than the maximum concentration in the absence of said anionic polymer, and said solution having a pH in the range within $\pm 2$ pH units of the isoelectric point of said protein and an ionic strength of 0.05 mole/l or less.

2.   An aqueous solution according to Claim 1, in which the anionic polymer or the salt thereof is present at a ratio to the protein of 1:40 - 100:1 by weight.

3.   An aqueous solution according to Claim 1 or 2, in which the presence of any salts other than the anionic polymer or the salt thereof is 0.1 mol or less per 1 mg of the protein.

4.   An aqueous solution according to Claim 1 or 2, in which the anionic polymer has at least one type of anion residue selected from carboxyl, carboxymethyl, sulfuric and phosphoric groups.

5.   An aqueous solution according to Claim 1 or 2, in which the protein is a physiologically active protein.

6.   An aqueous solution according to Claim 1 or 2, in which the isoelectric point of the protein is pH 4 or more.

7.   A method for increasing the concentration of protein in an aqueous solution comprising adding an anionic polymer or salt thereof to said solution, wherein the amount of said anionic polymer is sufficient to dissolve the protein to a concentration greater than the maximum concentration attainable in the absence of anionic polymer, and said solution has a pH within the range of $\pm 2$ pH units of the isoelectric point of the protein and an ionic strength of 0.05 mole/l or less.

8.   A method according to Claim 7, in which the protein is a physiologically active protein.

9.   A method according to Claim 7, in which the isoelectric point of the protein is pH 4 or more.

10. A pharmaceutical composition comprising an anionic polymer or salt thereof and a protein having poor solubility at a pH range around its isoelectric point, wherein the amount of said anionic polymer is sufficient to obtain an aqueous solution containing a greater amount of protein than the maximum amount of protein attainable in the absence of anionic polymer, the solution having a pH within the range of +2 pH units of the isoelectric point of the protein and an ionic strength of 0.05 mole/l or less.

11. A composition according to Claim 10, in which the protein is a physiologically active protein.

12. A composition according to Claim 10, in which the isoelectric point of the protein is pH 4 or more.

**Claims for the following Contracting States : ES, GR**

1. A method for increasing the concentration of protein in an aqueous solution comprising adding an anionic polymer or salt thereof to said solution, wherein the amount of said anionic polymer is sufficient to dissolve the protein to a concentration greater than the maximum concentration attainable in the absence of anionic polymer, and said solution has a pH within the range of +2 pH units of the isoelectric point of the protein and an ionic strength of 0.05 mole/l or less.

2. A method according to Claim 1, comprising adding the anionic polymer or the salt thereof at a ratio to the protein of 1:40 - 100:1 by weight.

3. A method according to Claim 1 or 2, in which the presence of any salts other than the anionic polymer or the salt thereof is kept at 0.1 mol or less per 1 mg of the protein.

4. A method according to Claim 1 or 2, in which the anionic polymer added to the solution has at least one type of anion residue selected from carboxyl, carboxymethyl, sulfuric and phosphoric groups.

5. A method according to Claim 1, in which the protein is a physiologically active protein.

6. A method according to Claim 1, in which the isoelectric point of the protein is pH 4 or more.

**Patentansprüche**

**Patentansprüche für folgende Vertragsstaaten : AT, BE, CH, DE, DK, FR, GB, IT, LI, LU, NL, SE**

1. Wässrige Lösung mit erhöhter Protein-Konzentration, umfassend dieses Protein und ein anionisches Polymer oder dessen Salz, wobei die Konzentration des Proteins in der Lösung größer ist als die maximale Konzentration in Abwesenheit des anionischen Polymers, und wobei die Lösung einen pH im Bereich von innerhalb +/- 2 pH-Einheiten vom isoelektrischen Punkt des Proteins und eine Ionenstärke von 0,05 Mol/l oder weniger aufweist.

2. Die wässrige Lösung nach Anspruch 1, bei der das anionische Polymer oder dessen Salz in einem Verhältnis zum Protein, bezogen auf das Gewicht, von 1:40 bis 100:1 vorliegt.

3. Die wässrige Lösung nach Anspruch 1 oder 2, bei der irgendwelche anderen Salze als das anionische Polymer oder dessen Salz in einer Konzentration von 0,1 Mol oder weniger pro 1 mg des Proteins anwesend sind.

4. Die wässrige Lösung nach Anspruch 1 oder 2, bei der das anionische Polymer wenigstens eine Sorte von anionischem Rest aufweist, der aus Carboxyl-, Carboxymethyl-, Schwefelsäure- und Phosphorsäure-Gruppen ausgewählt ist.

5. Die wässrige Lösung nach Anspruch 1 oder 2, bei der das Protein ein physiologisch aktives Protein ist.

6. Die wässrige Lösung nach Anspruch 1 oder 2, bei der der isoelektrische Punkt des Proteins einen pH-Wert von 4 oder mehr aufweist.

7. Verfahren zur Erhöhung der Protein-Konzentration in einer wässrigen Lösung, umfassend die Zugabe eines anionischen Polymers oder dessen Salzes zu dieser Lösung, wobei die Menge dieses anionischen Polymers ausreichend ist, um das Protein in einer Konzentration, die größer ist als die maximale, in Abwesenheit des anionischen Polymers erhältliche Konzentration, zu lösen, und wobei die Lösung einen pH im Bereich von innerhalb +/- 2 pH-Einheiten vom isoelektrischen Punkt des Proteins und eine Ionenstärke von 0,05 Mol/l oder weniger aufweist.

8. Das Verfahren nach Anspruch 7, bei dem das Protein ein physiologisch aktives Protein ist.

9. Das Verfahren nach Anspruch 7, bei dem der isoelektrische Punkt des Proteins einen pH-Wert von 4 oder mehr aufweist.

10. Pharmazeutische Zusammensetzung, umfassend ein anionisches Polymer oder dessen Salz und ein Protein mit einer geringen Löslichkeit in einem pH-Bereich um seinen isoelektrischen Punkt, wobei die Menge dieses anionischen Polymers ausreichend ist, um eine wässrige Lösung zu erhalten, die das Protein in einer Menge enthält, die größer ist als die maximale, in Abwesenheit des anionischen Polymers erhältliche Menge, und wobei die Lösung einen pH im Bereich von innerhalb +/- 2 pH-Einheiten vom isoelektrischen Punkt des Proteins und eine Ionenstärke von 0,05 Mol/l oder weniger aufweist.

11. Die Zusammensetzung nach Anspruch 10, bei der das Protein ein physiologisch aktives Protein ist.

12. Die Zusammensetzung nach Anspruch 10, bei der der isoelektrische Punkt des Proteins einen pH-Wert von 4 oder mehr aufweist.

**Patentansprüche für folgende Vertragsstaaten : ES, GR**

1. Verfahren zur Erhöhung der Protein-Konzentration in einer wässrigen Lösung, umfassend die Zugabe eines anionischen Polymers oder dessen Salzes zu dieser Lösung, wobei die Menge dieses anionischen Polymers ausreichend ist, um das Protein in einer Konzentration, die größer ist als die maximale, in Abwesenheit des anionischen Polymers erhältliche Konzentration, zu lösen, und wobei die Lösung einen pH im Bereich von innerhalb +/- 2 pH-Einheiten vom isoelektrischen Punkt des Proteins und eine Ionenstärke von 0,05 Mol/l oder weniger aufweist.

2. Das Verfahren nach Anspruch 1, umfassend die Zugabe des anionischen Polymers oder dessen Salzes in einem Verhältnis zum Protein, bezogen auf das Gewicht, von 1:40 bis 100:1.

3. Das Verfahren nach Anspruch 1 oder 2, bei dem irgendwelche anderen Salze als das anionische Polymer oder dessen Salz in einer Konzentration von 0,1 Mol oder weniger pro 1 mg des Proteins anwesend sind.

4. Das Verfahren nach Anspruch 1 oder 2, bei dem das anionische Polymer, das der Lösung zugesetzt wird, wenigstens eine Sorte von anionischem Rest aufweist, der aus Carboxyl-, Carboxymethyl-, Schwefelsäure- und Phosphorsäure-Gruppen ausgewählt ist.

5. Das Verfahren nach Anspruch 1, bei dem das Protein ein physiologisch aktives Protein ist.

6. Das Verfahren nach Anspruch 1, bei dem der isoelektrische Punkt des Proteins einen pH-Wert von 4 oder mehr aufweist.

**Revendications**

**Revendications pour les Etats contractants suivants : AT, BE, CH, DE, DK, FR, GB, IT, LI, LU, NL, SE**

1. Solution aqueuse d'une concentration accrue en protéine se composant de ladite protéine et d'un polymère anionique ou d'un de ses sels, la concentration de ladite protéine dans ladite solution étant supérieure à la concentration maximale en l'absence dudit polymère anionique, et ladite solution ayant un pH dans la gamme de ± 2 unités pH du point isoélectrique de ladite protéine et une force ionique de 0,05 mole/l ou inférieure.

2. Solution aqueuse selon la revendication 1, dans laquelle le polymère anionique ou son sel est présent à raison d'un rapport à la protéine de 1:40 à 100:1 en poids;

3. Solution aqueuse selon la revendication 1 ou 2, dans laquelle la présence de tout sel autre que le polymère anionique ou de son sel est de 0,1 mole ou moins pour 1 mg de protéine.

4. Solution aqueuse selon la revendication 1 ou 2, dans laquelle le polymère anionique possède au moins un type de résidu anionique choisi parmi les groupements carboxyle, carboxyméthyle, sulfurique et phosphorique.

5. Solution aqueuse selon la revendication 1 ou 2, dans laquelle la protéine est une protéine active sur le plan ph-physiologique.

6. Solution aqueuse selon la revendication 1 ou 2, dans laquelle le pH du point isoélectrique de la protéine est de 4 ou plus.

7. Procédé pour augmenter la concentration en protéine dans une solution aqueuse comprenant l'étape consistant à ajouter un polymère anionique ou un de ses sels à ladite solution, dans lequel la quantité dudit polymère anionique est suffisante pour dissoudre la protéine à une concentration supérieure à la concentration atteignable en l'absence de polymère anionique, et ladite solution possède un pH dans la gamme de ± 2 unités pH du point isoélectrique de la protéine et possède une force ionique de 0,05 mole/l ou moins.

8. Procédé selon la revendication 7, dans lequel la protéine est une protéine active sur le plan physiologique.

9. Procédé selon la revendication 7, dans lequel le pH du point isoélectrique de la protéine est de 4 ou plus.

10. Composition pharmaceutique comprenant un polymère anionique ou un de ses sels et une protéine possédant une solubilité médiocre à une gamme de pH aux environs de son point isoélectrique, dans laquelle la quantité dudit polymère anionique est suffisante pour obtenir une solution aqueuse contenant une quantité supérieure de protéine que la quantité maximale de protéine atteignable en l'absence de polymère anionique, la solution ayant un pH dans la gamme de ± 2 unités pH du point isoélectrique de la protéine et une force ionique de 0,05 mole/l ou inférieure.

11. Composition selon la revendication 10, dans laquelle la protéine est une protéine active sur le plan physiologique.

12. Composition selon la revendication 10, dans laquelle le pH du point isoélectrique de la protéine est de 4 ou plus.

**Revendications pour les Etats contractants suivants : ES, GR**

1. Procédé pour augmenter la concentration en protéine dans une solution aqueuse comprenant l'étape consistant à ajouter un polymère anionique ou un de ses sels à ladite solution, dans lequel la quantité dudit polymère anionique est suffisante pour dissoudre la protéine à une concentration supérieure à la concentration maximale atteignable en l'absence de polymère anionique, et ladite solution possède un pH dans la gamme de ± 2 unités pH du point isoélectrique de la protéine et possède une force ionique de 0,05 mole/l ou moins.

2. Procédé selon la revendication 1, comprenant l'étape consistant à ajouter le polymère anionique ou son sel à raison d'un rapport à la protéine de 1:40 à 100:1 en poids.

3. Procédé selon la revendication 1 ou 2, dans lequel on maintient la présence de tous sels autre que le polymère anionique ou de son sel à 0,1 mole ou moins pour 1 mg de protéine.

4. Procédé selon la revendication 1 ou 2, dans lequel le polymère anionique ajouté à la solution possède au moins un type de résidu anionique choisi parmi les groupements carboxyle, carboxyméthyle, sulfurique et phosphorique.

5. Procédé selon la revendication 1, dans lequel la protéine est une protéine active sur le plan physiologique.

6. Procédé selon la revendication 1, dans lequel le pH du point isoélectrique de la protéine est de 4 ou plus.